Europäisches Patentamt

**European Patent Office**

**Office européen des brevets**

(11) Publication number: **0 416 398 A1**

## EUROPEAN PATENT APPLICATION

(21) Application number: 90116250.3

(22) Date of filing: 24.08.90

(51) Int. Cl.⁵: **A61L 27/00, A61L 25/00**

(30) Priority: 24.08.89 JP 218280/89

(43) Date of publication of application:
**13.03.91 Bulletin 91/11**

(84) Designated Contracting States:
**DE NL SE**

(71) Applicant: **ASAHI KOGAKU KOGYO
KABUSHIKI KAISHA
36-9, Maeno-cho 2-Chome Itabashi-ku
Tokyo 174(JP)**

(72) Inventor: **Sumita, Masaya, c/o Asahi Kogaku
Kogyo K.K.
36-9, Maeno-cho 2-chome
Itabashi-ku, Tokyo(JP)**

(74) Representative: **Hansen, Bernd, Dr.
Dipl.-Chem. et al
Hoffmann, Eitle & Partner Patent- und
Rechtsanwälte Arabellastrasse 4
W-8000 München 81(DE)**

(54) Paste for bonding granular bone prosthesis and bone prosthesis using same.

(57) A paste for bonding a granular bone prosthesis
and a bone prosthesis using the paste are disclosed,
which paste comprises an aqueous solution containing at least one member selected from the group
consisting of pullulan, glycol chitin, carboxymethyl
chitin and pectin. The paste is capable of bonding
any kind of granular bone prostheses to themselves
without causing harm to a living body.

EP 0 416 398 A1

# PASTE FOR BONDING GRANULAR BONE PROSTHESIS AND BONE PROSTHESIS USING SAME

## FILED OF THE INVENTION

The present invention relates to a paste for bonding a granular bone prosthesis and to a bone prosthesis which comprises a granular bone prosthesis bonded by the paste.

## BACKGROUND OF THE INVENTION

Application of hydroxyapatite as a bone prosthesis for dental and medical use has been studied because of its excellent biocompatibility and osteoconductivity, and several kinds of bone prostheses using hydroxyapatite have been put into practical use. Bone prostheses include granules and premolded blocks. In particular, granular bone prostheses have been widely employed since they can be arbitrarily filled in a deficient part of any shape.

However, granular bone prostheses lack bonding properties to themselves and are easily scattered and lost before they adhere to new bone. In order to overcome this disadvantage, it has been attempted to use fibrin as a paste for bonding the granules to each other as described in JP-A-60-254640 and JP-A-60-25641 (the term "JP-A" as used herein means an "unexamined published Japanese patent application"). A fibrin paste, however, raises a fear of infection with hepatitis virus, AIDS virus, etc. because it is prepared from human blood.

The present inventors previously proposed a process for bonding granules containing α-tricalcium phosphate or tetracalcium phosphate as an essential component with an acid aqueous solution as disclosed in JP-A-2-1285 and EP-A-0324425. However, this technique is only applicable to granules comprising a certain specific component.

## SUMMARY OF THE INVENTION

An object of the present invention is to provide a paste for bonding granular bone prostheses for medical and dental use of any type, which does not cause harm (e.g., infection) to a living body.

Another object of the present invention is to provide a bone prosthesis comprising a granular bone prosthesis which has excellent bonding properties to the granules thereof so that the granules are not scattered and lost before or after filling in a deficient part.

Other objects and effects of the present invention will be apparent from the following description.

The present invention therefore relates to a paste for bonding a granular bone prosthesis which comprises an aqueous solution containing at least one member selected from the group consisting of pullulan, glycol chitin, carboxymethyl chitin and pectin.

The present invention further relates to a bone prosthesis comprising a granular bone prosthesis which is bonded with the above-described paste.

## DETAILED DESCRIPTION OF THE INVENTION

One or more of the pullulan, glycol chitin, carboxymethyl chitin and pectin is/are dissolved in water to form a high viscosity solution having tackiness. The aqueous solution according to the present invention preferably has a viscosity of at least 1,000 cps, more preferably at least 2,000 cps, at room temperature (25°C). If the viscosity is less than 1,000 cps, the tackiness of the solution is insufficient, and the granules cannot be sufficiently bonded due to too high a fluidity of the solution. The upper limit of the viscosity is not particularly limited, but depends on the solubility of the pullulan, glycol chitin, carboxymethyl chitin and pectin because the higher concentration is required for attaining the higher viscosity.

Since pullulan, glycol chitin, carboxymethyl chitin and pectin do no harm in a living body, the paste of the present invention is highly safe upon use. Among these, pullulan and carboxymethyl chitin are preferably used in the present invention.

The properties such as the molecular weight of the pullulan, glycol chitin, carboxymethyl chitin and pectin vary depending on the production process thereof and are not particularly limited in the present invention. The concentration of the pullulan, glycol chitin, carboxymethyl chitin and pectin in the solution of the present invention is not particularly limited. However, the properties, such as the molecular weight, and the concentration are preferably selected to attain the above-recited viscosity of the solution.

In the preparation of the paste according to the present invention, for example, one or more of pullulan, glycol chitin, carboxymethyl chitin and pectin is/are first sterilized. The method of sterilization is not particularly restricted, but conventional high pressure steam sterilization (autoclaving) is preferred. The thus sterilized component is then dissolved in sterilized distilled water to obtain the paste of the present invention having the above-recited viscosity.

It is also possible to sterilize a paste prepared from a non-sterilized starting material. In this case, the paste can be sterilized by filtration or autoclaving. Sterilization by filtration is preferably carried out by utilizing gas pressure or by means of a pump, but autoclaving is preferred because of the high viscosity of the solution. The filtration is generally carried out by using a filter having a pore diameter of 0.5 μm or less. The autoclaving is generally carried out at a temperature of from 115 to 132°C and a pressure of from 0.7 to 2 kg/cm² for 10 to 30 minutes.

Upon production of the paste, such as autoclaving, care should be taken so that the temperature is less than 200°C. Should the temperature be 200°C or higher, there is the problem that the viscosity may be decreased.

The thus prepared paste can be mixed and kneaded with a granular bone prosthesis and filled in a deficient part of bone. Alternatively, the paste can be added to the granular bone prosthesis already filled in a deficient part of bone to bond the granules with each other.

The ratio of the paste to the granular bone prosthesis (paste/granular bone prosthesis) is preferably 1/2 or more. If the amount of the paste is too small, the granules may not be sufficiently bonded.

The paste of the present invention exhibits excellent tackiness as stated above and is therefore capable of bonding any kind of granular bone prosthesis among the granules thereof to thereby prevent scattering and loss of the granules.

The bone prosthesis according to the present invention comprises a granular bone prosthesis which are bonded to each other with the above-described paste.

The granular bone prosthesis which can be used in the present invention are not particularly limited, and any of granular bone prostheses generally used in the art can be used. Examples thereof are described, e.g., in U.S. Patents 4,693,986, 4,097,935 and 4,629,464. The diameter of the granules may be from 100 μm to 6 mm, and the granules may be either dense or porous. Examples of the material for the granules include calcium phosphate ceramic granules, alumina ceramic granules and zirconia ceramic granules. Preferred of them are calcium phosphate ceramic granules comprising at least one of hydroxyapatite, fluorapatite, α-tricalcium phosphate, β-tricalcium phosphate and tetracalcium phosphate. Among these, hydroxyapatite and tricalcium phosphate are particularly preferred in view of biocompatibility.

These granules can be obtained by various conventional processes as described e.g., in U.S.

Patents 4,693,986, 4,097,935 and 4,629,464, for example, a granulation process by high-speed stirring, grinding a compressed powder, and grinding a cake prepared by a conventional wet process. If desired, the thus prepared granules may be calcined, or a calcined raw material may be granulated. Further, the shape of the granules is not particularly limited.

The present invention is now illustrated in greater detail with reference to Examples, but it should be understood that the present invention is not deemed to be limited thereto.

EXAMPLE 1

9 g of pullulan ("PI-20" produced by Hayashibara K.K., molecular weight: about 20,000) was subjected to autoclaving at 121°C under a pressure of 1 kg/cm² and dissolved in 50 g of sterilized distilled water to prepare a paste. The resulting paste had a viscosity of 2,000 cps at room temperature.

When 1 g of the paste was kneaded with commercially available granular bone prosthesis ("Apaceram G" produced by Asahi Kogaku Kogyo Kabushiki Kaisha, hydroxyapatite granules having a diameter of from 300 to 600 μm), the granules were bonded among themselves with the paste and were hardly capable of being scattered.

EXAMPLE 2

18 g of pectin ("HM-1" produced by Mero Rousselot Satia, esterification degree: 74±2%, pH of 1% aqueous solution: 2.8-3.5) was subjected to autoclaving at 132°C and at a pressure of 2 kg/cm² and dissolved in 200 g of sterilized distilled water to prepare a paste. In order to accelerate dissolving, the system was heated in a microwave for domestic use for 5 minutes. The resulting paste had a viscosity of 14,000 cps at room temperature.

When 1 g of the paste was kneaded with 2 g of a commercially available granular bone prosthesis ("Apaceram G"), the granules were bonded to themselves with the paste and were hardly capable of being scattered.

EXAMPLE 3

20 mg of glycol chitin (produced by Katokichi Co., pH of aqueous solution: 6.5-7.5) was subjected to autoclaving at 121°C and at a pressure of 1 kg/cm² and dissolved in 1 g of sterilized distilled water to prepare a paste.

A calcium phosphate slurry was prepared by a conventional wet method in which an aqueous solution of phosphoric acid was added dropwise to a calcium hydroxide slurry. The calcium phosphate slurry obtained was powdered by spray drying and formed into a porous cake by a conventional hydrogen peroxide foaming method. The cake obtained was fired at 1,100°C for 4 hours and pulverized, followed by classification into a diameter of from 100 to 250 μm to thereby obtain porous granules. The granules were biphasic calcium phosphate (BCP) composed of 80 wt% of β-tricalcium phosphate and 20 wt% of hydroxyapatite based on the weight of the granules.

When 40 mg of the paste was kneaded with 30 mg of the granules, the granules were bonded among themselves by the paste and could hardly be scattered.

### EXAMPLE 4

The same procedures as in Example 3 were repeated except that the glycol chitin was replaced by carboxymethyl chitin ("CM-Chitin 100" produced by Katokichi Co., substitution degree: 1.0, pH of aqueous solution: 6.5-7.5), and the granules having a diameter of from 100 to 250 μm were replaced by granules having a diameter of from 300 to 600 μm. The granules were bonded among themselves with the paste and could hardly be scattered. The viscosity of the paste was 10,000 cps at room temperature.

As described above, the paste for bonding granules according to the present invention involves neither the danger of infection associated with blood components nor harm to a living body and, therefore, has high safety. Further, the paste of the invention can be applied to any kind of granular bone prostheses to bond them to each other. The granular bone prosthesis bonded to each other with the paste of the present invention can be fixed and retained in a place where desired without being scattered and lost.

While the invention has been described in detail and with reference to specific embodiments thereof, it will be apparent to one skilled in the art that various changes and modifications can be made therein without departing from the spirit and scope thereof.

## Claims

1. A paste for bonding a granular bone prosthesis which comprises an aqueous solution containing at least one member selected from the group consisting of pullulan, glycol chitin, carboxymethyl chitin and pectin.

2. A paste as claimed in claim 1, wherein said aqueous solution has a viscosity of 1,000 cps or more at room temperature.

3. A paste as claimed in claim 2, wherein said aqueous solution has a viscosity of 2,000 cps or more at room temperature.

4. A bone prosthesis comprising a granular bone prosthesis which is bonded by a paste comprising an aqueous solution containing at least one member selected from the group consisting of pullulan, glycol chitin, carboxymethyl chitin and pectin.

5. A bone prosthesis as claimed in claim 4, wherein said granular bone prosthesis comprises calcium phosphate ceramic granules comprising at least one of hydroxyapatite, fluorapatite, α-tricalcium phosphate, β-tricalcium phosphate and tetracalcium phosphate.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 323 632 (ASAHI KOGAKU KOGYO) <br> * Page 6, lines 28-40; claims * | 1-5 | A 61 L 27/00 <br> A 61 L 25/00 |
| X | PATENT ABSTRACTS OF JAPAN, vol. 13, no. 466 (C-646)[3814], 20th October 1989; <br> & JP-A-1 181 872 (UNITIKA LTD) 19-07-1989 <br> * Abstract * | 1,5 | |
| X | US-A-3 502 466 (R.C. VICKERY) <br> * Column 3, lines 1-25; claims * | 1 | |
| D,Y | EP-A-0 324 425 (ASAHI KOGAKU KOGYO K.K.) <br> * Claims * | 1-5 | |
| Y | EP-A-0 298 501 (ASAHI KOGAKU KOGYO K.K.) <br> * Claims * | 1-5 | |
| A | WORLD PATENTS INDEX LATEST, week 30, accession no. 88-208078, Derwent Publications Ltd, London, GB; <br> & JP-A-63 143 071 (M. NAGASE) 15-06-1988 | | |

| TECHNICAL FIELDS SEARCHED (Int. Cl.5) |
|---|
| A 61 L |

The present search report has been drawn up for all claims

| Place of search | Date of completion of search | Examiner |
|---|---|---|
| The Hague | 29 November 90 | ESPINOSA Y CARRETERO |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document